# EUROPEAN PATENT APPLICATION

(11) **EP 4 043 008 A1**
(43) Date of publication of application: **17.08.2022**
(21) Application number: 21460013.2
(22) Date of filing: 15.02.2021
(51) Int. Cl.: A61K 9/19, A61K 9/00, A61K 9/10, A61K 31/496, A61P 25/18

(54) **METHOD FOR THE PREPARATION OF A PHARMACEUTICAL COMPOSITION COMPRISING ARIPIPRAZOLE**

(71) Applicant: Warszawskie Zaklady Farmaceutyczne Polfa S.A., 02-315 Warsaw (PL)
(72) Inventor: Kubisiak, Marcin, 02-315 Warsaw (PL); Pietrzak, Tomasz, 02-315 Warsaw (PL); Ratajczak, Tomasz, 02-315 Warsaw (PL); Szendzielorz, Ziemowit, 02-315 Warsaw (PL); Borychowska, Joanna, 02-315 Warsaw (PL)
(74) Representative: Kondrat, Mariusz

(57) **Abstract**

It relates to a process for the preparation of an extended-release pharmaceutical composition comprising aripiprazole and a pharmaceutically acceptable vehicle, wherein the process comprises: a) providing aripiprazole; b) providing a vehicle which is an aqueous solution, wherein the vehicle comprises a suspending agent which comprises a cellulose, and wherein the vehicle has been submitted to a thermal treatment; c) combining a) and b) to form a primary suspension comprising aripiprazole in the form of particles; and d) reducing the size of the aripiprazole particles to obtain a secondary suspension, wherein the aripiprazole particles in the secondary suspension have a volume moment mean diameter D[4,3] from 1 to 10 microns. The obtained suspension may be freeze-dried and stored and can be conveniently reconstituted as a suspension before administration.

## Description

### Technical Field

The present invention relates to a process for the preparation of an extended-release pharmaceutical composition comprising aripiprazole, and, in particular, an injectable aripiprazole suspension which may be obtained upon reconstitution of a freeze-dried formulation.

### Background Art

Aripiprazole is an atypical antipsychotic which exerts both agonistic and antagonistic activity at dopaminergic and serotonergic receptors and is used in the treatment of schizophrenia and bipolar illness. Its chemical name is 7-{4-[4-(2,3-dichlorophenyl)-1-piperazinyl]-butyloxy}-3,4-dihydro- 2(1 H)-quinolinone and has the following structure:

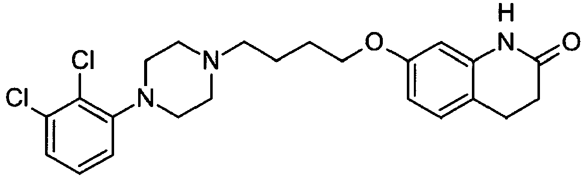

Aripiprazole is marketed under the tradename Abilify Maintena as powder and solvent for prolonged-release suspension for injection. The sterile lyophilized powder is to be reconstituted with water for injections to obtain an extended-release injectable suspension. In the hand of the present inventors, the volume moment mean diameter D[4,3] of aripiprazole in the aqueous vehicle is between 4.0 and 4.7 microns.

Methods for preparing sterile aripiprazole freeze-dried formulations are known in the art. For example, WO2005041937 discloses a method for preparing a sterile aripiprazole freeze-dried formulation which comprises: (a) preparing a sterile bulk aripiprazole having a mean particle size within the range from 5 to 100 microns, (b) preparing a sterile vehicle, (c) combining the sterile bulk aripiprazole and the sterile vehicle to form a sterile primary suspension, (d) reducing the mean particle size of aripiprazole in the primary suspension to a particle size within the range from 1 to 30 microns to form a final sterile suspension, and (e) freeze drying the final suspension. In example 1 of WO2005041937 the bulk aripiprazole is mixed with the sterile vehicle and homogenized under vacuum, then wet milled, and finally freeze dried. In example 2, aripiprazole (prepared by impinging jet crystallization) is mixed with the vehicle under vacuum, and then freeze dried. There is no indication in this document concerning the sterilization method used for the vehicle. The method described in this patent has some drawbacks to be applied on an industrial scale. First, bulk aripiprazole having a small particle size (less than 100 microns) and narrow particle size distribution is needed, which makes the process not versatile enough. Secondly, it requires complex steps such as mixing under vacuum and/or particular crystallization techniques such as impinging jet crystallization.

WO2009017250 describes a process for producing an aripiprazole suspension which comprises: (a) combining sterile bulk aripiprazole with a mean particle size of 200 to 400 microns and a vehicle which has been sterilized by filtration through a filter, to form a sterile primary suspension; (b) subjecting the primary suspension to first pulverization to form a sterile secondary suspension, preferably by using a high pressure homogenizer; and (c) subjecting the secondary suspension to second pulverization using a high-pressure homogenizer (up to 1000 bar, i.e. 100000 KPa) to form a sterile final suspension; wherein the aripiprazole in the final suspension has a mean particle size of 1 to 10 microns. The final suspension may be freeze dried. The process described in this document gives the best results for a very specific starting aripiprazole, which as mentioned above is not versatile. Besides, the method is also disadvantageous because it uses high-pressure homogenization at least in one step of the process, which is costly, imposes risks associated with operation of high pressure devices and possibly makes it hard to scale.

WO2012169662 discloses a freeze-dried aripiprazole formulation obtained by a process comprising: (a) preparing sterile bulk aripiprazole having a mean particle size of 5 to 1000 microns; (b) preparing a sterile vehicle for the sterile bulk aripiprazole; (c) mixing the aripiprazole and the vehicle to form a sterile primary suspension; (d) reducing the mean particle size of the aripiprazole in the primary suspension to the range of 1 to 10 microns to form a sterile final suspension; and (e) spraying for freezing the final suspension, and drying. According to this patent, impinging jet crystallization method and aseptic crystallization method are preferably used to produce bulk sterile aripiprazole. The particle size of the aripiprazole is reduced by wet milling, preferably by a high-pressure homogenizer method (550 bar, i.e. 55000 KPa) as shown in the examples. There is no indication in this document concerning the sterilization method used for the vehicle.

In summary, the methods of the prior art as disclosed above involve either complex and/or costly processing steps, and/or require starting from bulk aripiprazole having a small particle size. Consequently, there is still the need of providing improved methods which use conventional and robust techniques and allow obtaining aripiprazole extended-release formulations reproducibly.

### Summary of Invention

The present invention relates to processes for the preparation of extended-release pharmaceutical compositions comprising aripiprazole in the form of particles with a volume moment mean diameter D[4,3] from 1 to 10 microns, and a pharmaceutically acceptable vehicle. As it will be illustrated in the examples, the processes of the invention have the advantage that they are more versatile than the prior art methods since they are not very sensitive to the type of starting material and its particle size distribution. Thus, even bulk aripiprazole having a large particle size and/or a broad particle size distribution, which it is often much easier and cheaper to obtain can be used to produce a final product with a desired particle size distribution.

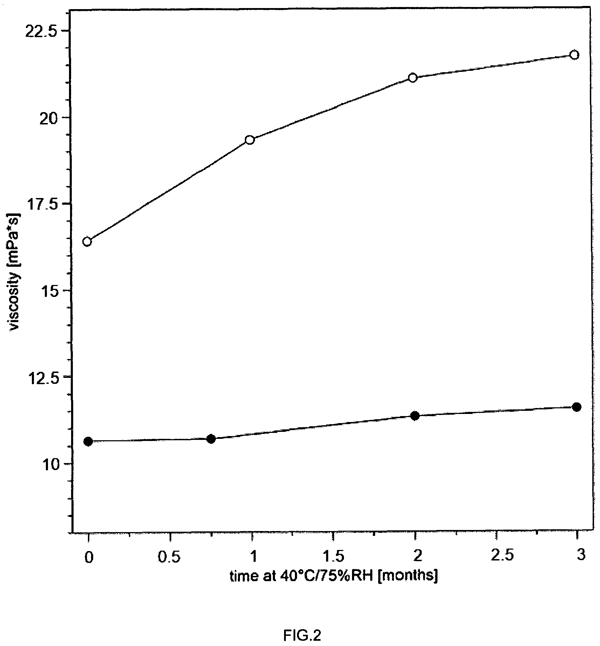

The inventors have found that when the vehicle comprising a suspending agent which comprises a cellulose, in particular celluloses sensitive to heating such as sodium carboxymethylcellulose, is submitted to a thermal treatment for its sterilization before combining it with aripiprazole, a final lyophilized product can be obtained which shows improved stability during shelf life. As can be seen in FIG. 2, the product that includes the thermal treatment in its preparation process (black circles) shows low viscosity values (around 10.5 mPa·s) which are maintained without any significant variation over time. By contrast, a product where the vehicle has not been sterilized by a thermal treatment (empty circles) shows not only much higher viscosity values (around 16.5 mPa·s) initially, but also the viscosity values very much increase over time (the viscosity is around 21.5 mPa·s after 3 months), which is undesirable from a stability point of view.

Although thermal sterilization is generally known in the art for sterilizing pharmaceutically acceptable vehicles, this method is not equally useful for every type of vehicle. For example, for a vehicle comprising a suspending agent which comprises a cellulose, in particular celluloses sensitive to heating such as sodium carboxymethylcellulose, thermal treatment for sterilization would be thought to be unsuitable *a priori,* given that it has been described that sodium carboxymethylcellulose suffers degradation (depolymerization by hydrolysis) and as a result undesired reduction of viscosity upon treatment at temperatures above 100 °C. In fact, in all the prior art documents describing aripiprazole formulations cited herein, the vehicle containing a suspending agent such as sodium carboxymethylcellulose is not subjected to thermal sterilization, but sterile filtration is the only method disclosed.

Unexpectedly, the inventors have found that in the present case the thermal treatment of the vehicle containing a suspending agent such as sodium carboxymethylcellulose did not significantly change the molecular weight of the carboxymethylcellulose, and rather provided an improved aripiprazole formulation in terms of stability. Without being bound to theory it is thought that the thermal sterilization may result in disentanglement or rearrangement of cellulose chains in space without degradation which may increase their availability to stabilize the active pharmaceutical ingredient particles. Further, thermal sterilization would apparently lead to lesser ability of the entire system to agglomerate (in solid state or after reconstitution), which is evidenced by lowered tendency of the suspension to increase its viscosity. Finally, thermal sterilization is considered the most reliable sterilization method.

Therefore, a first aspect of the invention relates to a process for the preparation of a pharmaceutical composition comprising aripiprazole and a pharmaceutically acceptable vehicle, wherein the process comprises:
a) providing aripiprazole;
b) providing a vehicle which is an aqueous solution, wherein the vehicle comprises a suspending agent which comprises a cellulose, and wherein the vehicle has been submitted to a thermal treatment;
c) combining a) and b) to form a primary suspension comprising aripiprazole in the form of particles; and
d) reducing the size of the aripiprazole particles to obtain a secondary suspension, wherein the aripiprazole particles in the secondary suspension have a volume moment mean diameter D[4,3] from 1 to 10 microns.

The present inventors have also found that improved stability of the final aripiprazole formulation is obtained when in the process for its preparation the reduction of the particle size of aripiprazole in a suspension containing aripiprazole and a vehicle is carried out by milling in recirculation with simultaneous homogenization instead of subjecting it simply to a milling step or a homogenizing step as disclosed for example in WO2005041937.

Thus, combining milling and homogenization as described herein improves homogeneity and results in reduction of particle size for aripiprazole since it prevents the formation of aggregates during milling.

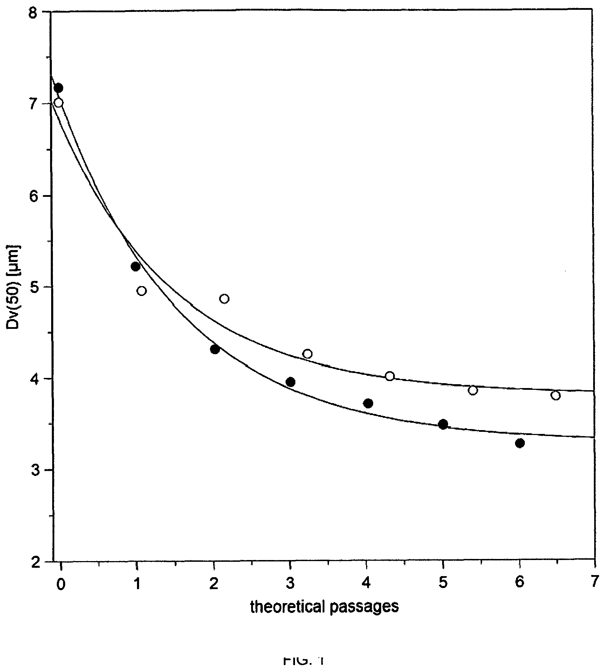

This can be seen in FIG.1 when comparing the Dv(50) drop for a batch where only milling is carried out (empty circles) and a batch using the combined milling and homogenization treatment of the present invention (black circles), which is indicative of disruption of active pharmaceutical ingredient agglomerates formed during milling. The combined milling and homogenization treatment also gives rise to a reduction in viscosity in the final product, which, without being bound to theory, it is supposed to be an effect of disaggregation.

By minimizing the amount of particle aggregates it is also ensured that unnecessary amounts of smallest particles which have a natural tendency to aggregate together or with larger particles to minimize their surface energy and also tend to dissolve in any available solvent and recrystallize on larger particles (Ostwald ripening), which adds instability of particle size and aggregation state, are produced. Besides, this step, which can be carried out by using a conventional mill and homogenizer, also allows to shorten the time of the process.

Thus, a second aspect of the invention relates to a process for the preparation of the pharmaceutical composition comprising aripiprazole and a vehicle which comprises:
i) providing aripiprazole;
ii) providing a vehicle which is an aqueous solution;
iii) combining i) and ii) to form a primary suspension comprising aripiprazole;
iv) reducing the size of the aripiprazole particles to obtain a secondary suspension,
wherein the aripiprazole particles in the secondary suspension have a volume moment mean diameter D[4,3] from 1 to 10 microns, wherein the reduction of the particle size comprises simultaneous milling and homogenization of the suspension of the previous step until the aripiprazole in the suspension has the desired particle size.

Especially good results are obtained when both techniques are combined, the thermal treatment and the combined milling and homogenizing treatment.

### Brief Description of Drawings

FIG. 1 shows the particle size decrease (Dv(50)) during the milling process for batches according to example 1 (black circles) and to comparative example 3 (empty circles).
FIG. 2 shows the viscosity of reconstituted freeze-dried suspensions for batches according to example 1 (black circles) and to comparative example 3 (empty circles).

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims.

The term "about" or "around" as used herein refers to a range of values ± 10% of a specified value. For example, the expression "about 10" or "around 10" includes ± 10% of 10, i.e. from 9 to 11.

As mentioned above, the present invention relates to processes for the preparation of extended-release pharmaceutical compositions comprising aripiprazole.

In the processes of the invention bulk aripiprazole is used as starting material. There is no limitation in the polymorphic form used for the bulk aripiprazole. For example, anhydrous, hydrated crystalline forms or mixtures thereof may be used, such as the ones selected from the group consisting of aripiprazole hydrate A, aripiprazole anhydrate B, aripiprazole anhydrate C, aripiprazole anhydrate D, aripiprazole anhydrate E, aripiprazole anhydrate F, and aripiprazole anhydrate G. These crystalline forms, as well as methods for their preparation are well-known in the art and are disclosed for example in the document WO2003026659.

In one embodiment, optionally in combination with one or more features of the various embodiments described above or below, the bulk aripiprazole is in the form of hydrate A as described in WO2003026659.

In a particular embodiment, the bulk aripiprazole is in the form of hydrate A and has a powder x-ray diffraction spectrum characterized by the following powder x-ray diffraction peaks at 2θ = 12.6, 15.4, 17.3, 18.0, 18.6, 22. 5 and 24.8. More particularly, the hydrate A has the powder x-ray diffraction diagram shown in Figure 3 of WO2003026659.

In another particular embodiment, the bulk aripiprazole is in the form of hydrate A and has infrared absorption bands at 2951, 2822, 1692, 1577, 1447, 1378, 1187, 963 and 784 cell on the IR (KBr) spectrum. In another particular embodiment, the bulk aripiprazole is in the form of hydrate A and has an ¹H-NMR spectrum (DMSO-d₆, TMS) having characteristic peaks at 1.55-1. 63 ppm (m, 2H), 1.68-1. 78 ppm (m, 2H), 2.35-2. 46 ppm (m, 4H), 2.48-2. 56 ppm (m, 4H + DMSO), 2.78 ppm (t, J = 7.4 Hz, 2H), 2.97 ppm (brt, J = 4.6 Hz, 4H), 3.92 ppm (t, J = 6.3 Hz, 2H), 6.43 ppm (d, J = 2.4 Hz, 1H), 6.49 ppm (dd, J = 8.4 Hz, J = 2.4 Hz, 1H), 7.04 ppm (d, J = 8.1 Hz, 1H), 7.11-7. 17 ppm (m, 1H), 7.28-7. 32 ppm (m, 2H) and 10.00 ppm (s, 1H). More particularly, the hydrate A has the ¹H-NMR spectrum (DMSO-d₆, TMS) shown in Figure 2 of WO2003026659.

In another particular embodiment, the bulk aripiprazole is in the form of hydrate A and has the thermogravimetric/differential thermogram shown in Figure 1 of WO2003026659.

Aripiprazole is generally present in the primary suspension in an amount within the range from 5 to 25% by weight, particularly from 7 to 20% by weight with respect to the total weight of the primary suspension. In the final suspension to be administered to the patient (reconstituted suspension from freeze-dried formulation with water), aripiprazole is generally present in an amount within the range from 1 to 40% by weight, particularly from 5 to 35% by weight, more particularly from 10 to 30% by weight, with respect to the total composition weight.

In one embodiment, optionally in combination with one or more features of the various embodiments described above or below, aripiprazole is present in the primary suspension in an amount within the range from 5 to 25% by weight, particularly from 7 to 20% by weight with respect to the total weight of the primary suspension.

The processes of the present invention have the advantage that they may use bulk aripiprazole having a broad range of particle size. Thus, it is not required to use bulk aripiprazole of small particle size of narrow particle size distribution, although it may be used if desired.

The particle size values indicated throughout this document may refer either to dry particles (D[4,3] in the case of the bulk aripiprazole) or to particles in suspension (D[4,3] and Dv(X) of aripiprazole particles in suspension). The particle size may be measured by laser diffraction, for example, a Malvern laser diffraction unit, Malvern Mastersizer 3000 particle size analyzer, from Malvern Instruments Ltd., Malvern, England may be employed.

When the particle size of aripiprazole is measured on dry particles (e.g. bulk aripiprazole), a Sirocco dispersing utility may be used.

As indicated above, the vehicle of the compositions disclosed herein is an aqueous solution. This means that all pharmaceutically acceptable excipients which from part of the vehicle are solubilized in water in the amounts in which they are used. Said amount will depend on the type of excipient. The term "solubilized" thus means that each excipient is present in the vehicle in an amount in which when contacting it with water it forms a solution. Since all excipients are solubilized in water, the particle size of aripiprazole may also be measured in a suspension comprising aripiprazole particles and the aqueous vehicle comprising the solubilized excipients, such as the primary suspension, the secondary suspension, the final suspension, or the reconstituted suspension). When the particle size of aripiprazole in a suspension is measured the method comprises: 1) Diluting the suspension to be analyzed with water for injections to make a predispersion; 2) Adding the predispersion to the dispersing utility of Mastersizer 3000 (MV or EV) to obtain required laser obscuration; 3) Apply sonication for specified amount of time and with specified intensity to disrupt any aggregates formed during resuspension or predispersion preparation; 4) Starting measurement using standard technique; and 5) Presenting results using Mie theory for non-spheric particles.

In one embodiment, optionally in combination with one or more features of the various embodiments described above or below, the bulk aripiprazole is in the form of particles having a volume moment mean diameter D[4,3] from 50 to 200 microns. The particle size of bulk aripiprazole is measured as a solid as described above.

In one embodiment, optionally in combination with one or more features of the various embodiments described above or below, the pharmaceutical compositions prepared in the present invention are sterile. The term "sterile" means that it has been undergone a sterilization process. Sterilization is the process of deactivation or removal of all forms of life and/or other biological agents present. For the purposes of the invention, the terms "sterile" or "sterilized" composition are used herein interchangeably and refer to the compound, vehicle or composition that does not present microbial and/or fungal contamination.

In one embodiment, optionally in combination with one or more features of the various embodiments described above or below, the bulk aripiprazole is sterile, more particularly the bulk aripiprazole has been sterilised by filtration.

The bulk aripiprazole is combined with a pharmaceutically acceptable vehicle to form a primary suspension. The expression "pharmaceutically acceptable vehicle" as used herein refers to materials, excipients or compositions which are pharmaceutically acceptable, where any of the individual components must be compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

As mentioned above, the excipients used in the pharmaceutical compositions of the present invention have to be soluble in water in the amounts in which they are employed.

In step b) of the first aspect of the invention a vehicle is provided which is an aqueous solution, wherein the vehicle comprises a suspending agent which comprises a cellulose, in particular a cellulose sensitive to heating, more particularly the suspending agent is present in an amount such that it is solubilized in water, i.e. forms an aqueous solution.

In one embodiment, optionally in combination with one or more features of the various embodiments described above or below, the vehicle further comprises one or more excipients selected from the group consisting of bulking agents, buffering agents, pH adjusting agents, and combinations thereof, more particularly wherein the excipients are present in amount such that they are solubilized in water, i.e. form an aqueous solution.

If desired, the vehicle may also comprise other suspending agents apart from celluloses.

The term "suspending agent" as used in the present invention refers to compounds that facilitate active pharmaceutical ingredients stay suspended in the formulation and prevent and/or reduce the phase separation. Non-limiting examples of suspending agents include carboxymethyl cellulose or a pharmaceutically acceptable salt thereof, hydroxypropyl cellulose, carboxymethyl cellulose, hydroxypropylethyl cellulose, hydroxypropylmethyl cellulose, and polyvinylpyrrolidone. Pharmaceutically acceptable salts of carboxymethyl cellulose include, without limitation, alkali metal salts, such as sodium carboxymethyl cellulose, potassium carboxymethyl cellulose, and lithium carboxymethyl cellulose, alkaline earth metal salts, such as magnesium carboxymethyl cellulose and calcium carboxymethyl cellulose, or ammonium carboxymethyl cellulose.
In the vehicle which is combined with the aripiprazole to form the primary suspension, the suspending agent is generally present in an amount within the range from 0.1 to 1.5% by weight, particularly from 0.15 to 0.8% by weight with respect to the total weight of the vehicle. In the final suspension to be administered to the patient (reconstituted suspension from freeze-dried formulation with water), the suspending agent is generally present in an amount within the range from 0.2 to 5% by weight, particularly from 0.3 to 2% by weight with respect to the total composition weight.

In one embodiment, optionally in combination with one or more features of the various embodiments described above or below, the vehicle comprises a suspending agent which comprises a cellulose, more particularly a cellulose which is sensitive to heating, and even more particularly, the cellulose sensitive to heating is selected from the group consisting of sodium, potassium or other carboxymethyl cellulose pharmaceutically acceptable salts, carboxymethyl cellulose, hydroxypropyl cellulose, carboxymethyl cellulose, hydroxypropylethyl cellulose, hydroxypropylmethyl cellulose, and mixtures thereof. The expression "cellulose which is sensitive to heating" as used herein means that its solution when exposed to heat exhibits irreversible loss of viscosity resulting significant from the point of view of the final product's quality. The suspending more particularly is carboxymethyl cellulose or a pharmaceutically acceptable salt thereof such as sodium carboxymethyl cellulose or a pharmaceutically acceptable salt thereof. More particularly, the suspending agent is present in the primary suspension in an amount within the range from 0.1 to 1.5% by weight, particularly from 0.15 to 0.80% by weight with respect to the total weight of the vehicle.

In one embodiment of the first aspect of the invention, optionally in combination with one or more features of the various embodiments described above or below, the vehicle comprises a suspending agent which comprises a cellulose, more particularly the cellulose is selected from the group consisting of sodium, potassium or other carboxymethyl cellulose pharmaceutically acceptable salts, carboxymethyl cellulose, hydroxypropyl cellulose, carboxymethyl cellulose, hydroxypropylethyl cellulose, hydroxypropylmethyl cellulose, and mixtures thereof, even more particularly, the cellulose is sodium carboxymethyl cellulose. In another embodiment, the suspending agent is carboxymethyl cellulose or a pharmaceutically acceptable salt thereof such as sodium carboxymethyl cellulose.

The term "bulking agent" as used herein refers to compounds that increase the volume or weight of the pharmaceutical formulation while keeping its functionality intact. A bulking agent may also contribute to the physical structure, uniformity, and stability of a freeze-dried formulation. Non-limiting examples of bulking agents include mannitol, sucrose, maltose, lactose, xylitol, and sorbitol. In the vehicle which is combined with the aripiprazole to form the primary suspension, the bulking agent is generally present in an amount within the range from 0.5 to 10% by weight, particularly from 1 to 8% by weight with respect to the total weight of the vehicle. In the final suspension to be administered to the patient (reconstituted suspension from freeze-dried formulation with water), the bulking agent is generally present in an amount within the range from 1 to 10% by weight, particularly from 2 to 8% by weight, with respect to the total composition weight.

In one embodiment, optionally in combination with one or more features of the various embodiments described above or below, the vehicle comprises a bulking agent, more particularly the bulking agent is selected from the group consisting of mannitol, sucrose, maltose, lactose, xylitol, and sorbitol, and more particularly is mannitol. More particularly, the bulking agent is present in the primary suspension in an amount within the range from 0.5 to 10% by weight, particularly from 1 to 8 by weight with respect to the total weight of the vehicle.

The term "buffering agent" as used herein refers to weak acids or bases used to maintain the pH of the aqueous formulation near a chosen pH value after the addition of another acid or base. Non-limiting examples of buffering agents include sodium phosphate, disodium phosphate, disodium phosphate monohydrate, potassium phosphate, and TRIS buffer (2-amino-2-hydroxymethyl-propane-1,3-diol). In the vehicle which is combined with the aripiprazole to form the primary suspension, the buffering agent is generally present in an amount within the range from 0.01 to 0.5% by weight, particularly from 0.02 to 0.1% by weight with respect to the total weight of the vehicle. In the final suspension to be administered to the patient (reconstituted suspension with water), the buffering agent is generally present in an amount within the range from 0.02 to 2% by weight, particularly from 0.03 to 1.5% by weight, with respect to the total composition weight.

In one embodiment, optionally in combination with one or more features of the various embodiments described above or below, the vehicle comprises a buffering agent, more particularly the buffering agent is selected from the group consisting of sodium phosphate, disodium phosphate, disodium phosphate monohydrate, potassium phosphate, and TRIS buffer, more particularly disodium phosphate monohydrate. More particularly, the buffering agent is present in the primary suspension in an amount within the range from 0.01 to 0.5% by weight, particularly from 0.02 to 0.1% by weight with respect to the total weight of the vehicle.

The term "pH adjusting agent" as used herein refers to a compound which is employed to adjust pH of the aqueous suspension within the range from 6 to 7.5, more particularly at about 7. When the pH needs to be lowered, the pH adjusting agent will be an acid such as, without limitation, hydrochloric acid or acetic acid. When the pH needs to be increased, the pH adjusting agent will be a base such as, without limitation, sodium hydroxide, potassium hydroxide, calcium carbonate, magnesium oxide or magnesium hydroxide.

In one embodiment, optionally in combination with one or more features of the various embodiments described above or below, the vehicle comprises a pH adjusting agent, more particularly the pH adjusting agent is selected from the groups consisting of hydrochloric acid, acetic acid, sodium hydroxide, potassium hydroxide, calcium carbonate, magnesium oxide or magnesium hydroxide, more particularly is sodium hydroxide. More particularly, the pH adjusting agent is present in the primary suspension in an amount sufficient to adjust the pH of the formulation within the range from 6 to 7.5.

In one embodiment, optionally in combination with one or more features of the various embodiments described above or below, the vehicle comprises a suspending agent, a bulking agent, a buffering agent, a pH adjusting agent, and water. More particularly, the vehicle comprises a suspending agent which is carboxymethyl cellulose or a pharmaceutically acceptable salt thereof such as sodium carboxymethyl cellulose, a bulking agent which is mannitol, a buffering agent which is disodium phosphate monohydrate, a pH adjusting agent which is sodium hydroxide, and water. Even more particularly, the vehicle of the primary suspension comprises from 0.10 to 1.5% by weight of a suspending agent such as sodium carboxymethyl cellulose, from 0.5 to 10% by weight of a bulking agent such as mannitol, from 0.01 to 0.5% of by weight a buffering agent disodium phosphate monohydrate, from 85 to 99% by weight of water, and, if needed, a pH adjusting agent such as sodium hydroxide in an amount sufficient to adjust the pH of the formulation within the range from 6 to 7.5, wherein all percentages are with respect to the total weight of the vehicle and wherein the sum of the percentages is equal to or lower than 100%.

In one embodiment, optionally in combination with one or more features of the various embodiments described above or below, the pH of the vehicle is from 6 to 7.5.

According to the first aspect of the invention the process comprises the step of b) of providing a vehicle, which has been submitted to a thermal treatment, in particular for its sterilization, whereby a thermally sterilized vehicle is obtained. Thus, in one embodiment, optionally in combination with one or more features of the various embodiments described above or below, step b) comprises providing a thermally sterilized vehicle.

For the purposes of the invention, the vehicle that has been submitted to a thermal treatment, in particular for its sterilization, is herein also referred to as thermally treated vehicle.

The term "thermally sterilized" or "heat sterilized" vehicle as used herein interchangeably and refer to the vehicle that has been undergone a thermal or heat sterilization. Thermal or heat sterilization uses the thermal lability of a microorganism to degrade its vital components and refers to the process of exposing the vehicle to elevated temperatures for sufficient time and temperature to kill and/or inactivate any microorganisms present to a level acceptable for the intended use.

The thermal treatment according to the present invention may be generally performed with dry heat in a dry heat chamber designed for that purpose, or by steam or moist heat for example in an autoclave or in a tank equipped with a heating jacket, or by any other device used to heat solutions. When dry heat is used, the process is referred to as dry heat treatment or dry heat sterilization and the material is said to have been dry heat-treated or dry heat-sterilized, and when steam heat is used, the process is referred to as steam treatment or steam sterilization and the material is said to have been steam-treated or steam-sterilized. Dry heat treatment is typically carried out at a temperature range from 160-170 °C for a period of time from 30-180 min. Steam treatment and heat treatment generally uses lower temperatures and durations than dry heat sterilization and is typically carried out at a temperature range from 110-130°C for a period of time from of 2 minutes to 2 hours. Other temperatures and times can be used for both steam sterilization and dry heat sterilization as known by those skilled in the art.

In one embodiment, optionally in combination with one or more features of the various embodiments described above or below, the thermal treatment to which the vehicle is submitted is a steam heat treatment, more particularly the vehicle is steam-sterilized, i.e. the thermally sterilized vehicle has undergone steam sterilization. More particularly, the steam heat treatment comprises placing the vehicle into an autoclave or in a tank equipped with a heating jacket, and applying steam heat at a temperature within the range from 110 to 130 °C, for a period of time from 2 minutes to 2 hours.

If desired, previously to the thermal treatment or after thermal treatment, the vehicle may be additionally sterilized through a filter, typically a 0.22 micron filter, although other filter sizes may be used as well. Thus, in one embodiment, optionally in combination with one or more features of the various embodiments described above or below, the vehicle is filtered through a filter, particularly a 0.22 micron filter, before being thermally treated or after that.

In step c) of the process of the first aspect, the bulk aripiprazole, particularly sterile, and the thermally treated vehicle are combined to form a primary suspension. In one embodiment, optionally in combination with one or more features of the various embodiments described above or below, the primary suspension is sterile.

It is of advantage that the primary suspension is homogenized. This way the particle size of aripiprazole already in suspension is decreased and may facilitate the subsequent step, in particular the combined milling and homogenization step which uses bead milling. Homogenization may be performed by using any type of homogenizer including a conventional homogenizer such a high shear homogenizer.

Thus, in one embodiment, optionally in combination with one or more features of the various embodiments described above or below, step c) further comprises homogenizing the primary suspension. More particularly, homogenizing is carried out with a high shear homogenizer at circumferential velocity from 1 to 100 m/s, more particularly from 15 to 40 m/s, for at least 15 min. The skilled person will easily know how to adjust the time needed depending on the specific equipment and volume of sample used.

In one embodiment, optionally in combination with one or more features of the various embodiments described above or below, homogenizing is carried out until the particle size of aripiprazole D[4,3] is from 10 to 40, more particularly from 10 to 20 microns, i.e. the particle size of aripiprazole D[4,3] in the primary suspension is from 10 to 40, more particularly from 10 to 20 microns. In another embodiment, optionally in combination with one or more features of the various embodiments described above or below, homogenizing is carried out until the particle size of aripiprazole particles Dv(100) is from 70 to 150, more particularly from 70 to 90 microns, i.e. the particle size of aripiprazole Dv(100) in the primary suspension is from 70 to 150, more particularly from 70 to 90 microns.

The term Dv(X), which can also be referred to as DX, is a value expressing the volume (v) median diameter and means that X% of the particles have a particle size below this value. Thus, for example, Dv(10) indicates the diameter where 10% of the particles of the sample has a smaller particle size, Dv(50) indicates the diameter where 50% of the particles of the sample has a smaller particle size, Dv(90) indicates the diameter where 90% of the particles of the sample has a smaller particle size, Dv(99) indicates the diameter where 99% of the particles of the sample has a smaller particle size, and Dv(100) indicates the diameter where 100% of the particles of the sample has a smaller particle size.

In step d) of the process of the first aspect of the invention the particle size of aripiprazole is reduced to obtain a secondary suspension, wherein the aripiprazole particles in the secondary suspension have a volume moment mean diameter D[4,3] from 1 to 10 microns. In one embodiment, optionally in combination with one or more features of the various embodiments described above or below, the secondary suspension is sterile.

Methods for reducing the particle size are well known in the art. These methods include for example milling, such as bead milling, where the primary suspension is subjected to grinding means in the presence of grinding media. It is advantageous to perform milling in recirculation with simultaneous homogenization. In a process comprising milling in recirculation with simultaneous homogenization, also referred herein to as "simultaneous milling and homogenization", "combined milling and homogenization", or "milling with online homogenization" the primary suspension is circulated between a homogenizer device and a milling device for one or more passes until the aripiprazole particles in the suspension have the desired particle size. This technique has the advantage that by combining milling and homogenization in the same step, the agglomerates that are continuously formed during the milling step are also continuously broken up in the homogenizer.

In one embodiment, optionally in combination with one or more features of the various embodiments described above or below, the reduction of the particle size of step d) comprises simultaneous milling and homogenization of the suspension of the previous step for one or more passes until the aripiprazole in the suspension has the desired particle size, i.e., a volume moment mean diameter D[4,3] from 1 to 10 microns.

Any milling technique may be used such as, for example, bead milling. As grinding media zirconium oxide beads in a mill such as LabStar LS1 or GammaVita from Netzsch. Wet milling comprises subjecting the suspension to mechanical means, such as a dispersion mill or grinding media, for reducing the size of the active pharmaceutical ingredient. One example of a dispersion mill is a bead mill. Milling beads may be made from polystyrene, glass, zirconium oxide stabilised with magnesia, zirconium oxide stabilised with yttrium, zirconium oxide stabilised with cerium, zirconium silicate, zirconia-alumina, stainless steel, titanium or aluminium. Particularly suitable are beads made of zirconium oxide stabilised with yttrium. Typically, the beads used are spherical and have mean diameter from 0.03 to 3.0 mm, for example 0.5 to 0.8 mm. The load of the beads in the mill chamber may be variable, for example a bead load from 30 to 90%, more particularly from 40 to 70% of nominal milling chamber volume may be used.

A separator is used to retain the beads within the mill chamber whilst allowing the passage of product out of the mill chamber. The pore size of the separator is chosen based on the milling beads diameter, generally the pore size of the screen is 3 times smaller than the diameter of the beads. For example, if milling beads of 0.5 mm diameter are used it is advantageous that the particle size of aripiprazole in the primary suspension is below 100 micron approximately. This can be conveniently achieved by carrying out a homogenization step previously to the simultaneous milling and homogenization as mentioned above.

Variable motor speeds and flowrates may be selected to optimise the milling process. For example, a typical mill circumferential velocity may be 2-20 m/s, and a typical flowrate may be 100-1000 g/min when the process is performed using milling chamber of 120 mL nominal volume. Further, the suspension may be passed through the bead mill just once (single pass) before being further processed, or a number of times (multiple passes). The best results are obtained when more than one milling passes are used. The number of passes refers to the probability that a given particle actually reached the milling chamber, and can be calculated by multiplying flowrate by time of milling and dividing by total amount of suspension in holding tank (homogenizer tank).

The skilled person will easily know how to adjust the milling parameters depending on the specific equipment and volume of sample used.

In one embodiment, optionally in combination with one or more features of the various embodiments described above or below, milling is performed with milling beads.

In one embodiment, optionally in combination with one or more features of the various embodiments described above or below, milling is bead milling, more particularly, wherein the milling beads are selected from the groups consisting of polystyrene, glass, zirconium oxide stabilised with magnesia, zirconium oxide stabilised with yttrium, zirconium oxide stabilised with cerium, zirconium silicate, zirconia-alumina, stainless steel, titanium or aluminium beads, more particularly, zirconium oxide stabilised with yttrium beads.

In one embodiment, optionally in combination with one or more features of the various embodiments described above or below, milling is carried out from 3 to 30 passes, more particularly from 5 to 8 passes, and even more particularly 6 to 7 passes.

In the simultaneous milling and homogenization step, homogenization may be performed by using any type of homogenizer including a conventional homogenizer such a high shear homogenizer. Thus, in one embodiment, optionally in combination with one or more features of the various embodiments described above or below, homogenization is carried out with a high shear homogenizer at a speed from 15 to 40 m/s for at least 30 min.

In one embodiment, optionally in combination with one or more features of the various embodiments described above or below, step d) of reducing the size of the aripiprazole particles, in particular the simultaneous milling and homogenizing step as described herein, is carried out until the particle size of aripiprazole D[4,3] in the secondary suspension is from 2 to 8, more particularly from 3 to 6 microns, i.e. the particle size of aripiprazole D[4,3] in the secondary suspension is from 2 to 8, more particularly from 3 to 6 microns. In another embodiment, optionally in combination with one or more features of the various embodiments described above or below, step d) of reducing the size of the aripiprazole particles, in particular the simultaneous milling and homogenizing step, is carried out until the particle size distribution of aripiprazole particles comprises Dv(10) from 1.0 to 1.6 microns, Dv(50) from 3.0 to 4.0 microns, Dv(90) from 6.0 to 12.0 microns, and Dv(99) from 15.0 to 40.0 microns, i.e. the particle size distribution of aripiprazole particles in the secondary suspension comprises Dv(10) from 1.0 to 1.6 microns, Dv(50) from 3.0 to 4.0 microns, Dv(90) from 6.0 to 12.0 microns, and Dv(99) from 15.0 to 40.0 microns.

If desired, after step d) of the process of the first aspect of the invention, a final homogenization step may be carried out. This final homogenization may ensure that all aggregates formed during milling are finally disrupted, and at the same time it may further equalize the distribution of the suspending agent, in particular carboxymethyl cellulose or a pharmaceutically acceptable salt thereof such as sodium carboxymethylcellulose, to cover the active pharmaceutical ingredient.

In one embodiment, optionally in combination with one or more features of the various embodiments described above or below, after step d) the process of the first aspect of the invention further comprises: e) homogenizing the secondary suspension to form a final suspension. More particularly, step e) is carried out with a high shear (rotor-stator) homogenizer at a speed from 15 to 40 m/s at least for 5 min.

In one embodiment, optionally in combination with one or more features of the various embodiments described above or below, the final suspension is sterile.

In one embodiment, optionally in combination with one or more features of the various embodiments described above or below, the particle size of aripiprazole D[4,3] in the final suspension obtained after step e) is from 2 to 8, more particularly from 3 to 6 microns. In another embodiment, optionally in combination with one or more features of the various embodiments described above or below, the particle size distribution of aripiprazole particles in the final suspension comprises Dv(10) from 1.0 to 1.6 microns, Dv(50) from 3.0 to 4.0 microns, Dv(90) from 6.0 to 12.0 microns, and Dv(99) from 15.0 to 40.0 microns.

If desired, the secondary suspension obtained in step d) or the final suspension of step e) may be finally freeze-dried, whereby the pharmaceutical composition is a freeze-dried aripiprazole formulation. In one embodiment, optionally in combination with one or more features of the various embodiments described above or below, the process of the first aspect of the invention further comprises freeze drying the suspension obtained in the previous step to form a freeze-dried formulation. More particularly, the freeze-dried formulation is sterile.

In one embodiment, optionally in combination with one or more features of the various embodiments described above or below, the particle size of aripiprazole D[4,3] in the freeze-dried formulation is from 1 to 10 microns, more particularly from 2 to 8, and even more particularly from 3 to 6 microns, measured in suspension after reconstitution. In another embodiment, optionally in combination with one or more features of the various embodiments described above or below, the particle size distribution of aripiprazole particles in the freeze-dried formulation comprises Dv(10) from 1.0 to 1.6 microns, Dv(50) from 3.0 to 4.0 microns, Dv(90) from 6.0 to 12.0 microns, and Dv(99) from 15.0 to 40.0 microns, measured in suspension after reconstitution.

Freeze drying, also called lyophilization, may be carried out in a lyophilizer such as Epsilon 2-6D LSCplus from Martin Christ by freezing the secondary or final suspension to a temperature from -20 to -60 °C particularly at about -40 °C. The temperature is typically stabilised in the range from -20 to -60 °C, typically at -35 °C, and the pressure is stabilised in the range 10 to 100 Pa, typically at 30 Pa. Before freezing, temperature stabilisation about 5 °C may be performed. After freezing the cooled suspension is dried at a temperature within the range from -10 to -30 °C, particularly at about -15 °C. Before the drying an annealing step may also be carried out at a temperature within the range from - 15 to -30° particularly at about -25 °C. Freeze drying may also include a secondary drying phase. After drying the temperature is raise in the range from -1 to 25 °C, typically at -5 °C, and the pressure is lowered to atmospheric using nitrogen to a final pressure in the range from 40 to 110 KPa.

The freeze-dried formulation may be reconstituted with water for injection before being administered to form a reconstituted suspension, which is able to release a therapeutic effective amount of aripiprazole over a period of at least one week, particularly two, three or four weeks, and up to six weeks or more.

The expression "therapeutically effective amount" as used herein, refers to the amount of aripiprazole which, when administered, is sufficient to prevent development of, or alleviate to some extent, one or more of the symptoms of the disease which is addressed. The specific dose of the compound of the invention to obtain a therapeutic benefit may vary depending on the particular circumstances of the individual patient including, among others, the size, weight, age and sex of the patient, the nature and stage of the disease, the aggressiveness of the disease, and the route of administration.

Thus, in one embodiment, optionally in combination with one or more features of the various embodiments described above or below, the process of the first aspect of the invention further comprises reconstituting the freeze-dried formulation obtained in the previous step in water, whereby the obtained pharmaceutical composition is a reconstituted suspension. More particularly, the reconstituted suspension is sterile.

In one embodiment, optionally in combination with one or more features of the various embodiments described above or below, the reconstituted suspension with water comprises aripiprazole in an amount from 1 to 40% by weight, particularly from 5 to 35% by weight, more particularly from 10 to 30% by weight, from 0.2 to 5% by weight of a suspending agent such as carboxymethyl cellulose or a pharmaceutically acceptable salt thereof such as sodium carboxymethyl cellulose, from 1 to 10% by weight of a bulking agent such as mannitol, from 0.02 to 2% of by weight a buffering agent disodium phosphate monohydrate, and a pH adjusting agent such as sodium hydroxide in an amount sufficient to adjust the pH of the formulation within the range from 6 to 7.5, wherein all percentages are with respect to the total weight of the composition, and wherein the sum of the percentages is equal to or lower than 100%.

In one embodiment, optionally in combination with one or more features of the various embodiments described above or below, the reconstituted suspension with water shows a viscosity value from 8 to 20 mPa·s, more particularly from 10 to 15 mPa·s. Viscosity may be measured using Brookfield type viscometer. In one embodiment, optionally in combination with one or more features of the various embodiments described above or below, the viscosity of the reconstituted suspension with water is maintained overtime, more particularly it does not significantly change in a period of 3 months, more particularly the change in viscosity in a period of 3 months does not change more than about 1.5 mPa·s.

As mentioned above, the first aspect of the invention relates to a process for the preparation of a pharmaceutical composition comprising aripiprazole and a pharmaceutically acceptable vehicle, wherein the process comprises steps a) to d), whereby the pharmaceutical composition is in the form of a suspension.

In one embodiment of the first aspect, the invention relates to a process for the preparation of a pharmaceutical composition comprising aripiprazole and a pharmaceutically acceptable vehicle, wherein the process comprises:
a) providing aripiprazole, particularly, aripiprazole in the form of particles having a volume moment mean diameter D[4,3] from 50 to 200 microns;
b) providing a vehicle which is an aqueous solution, wherein the vehicle comprises a suspending agent which comprises a cellulose, and wherein the vehicle has been submitted to a thermal treatment, more particularly wherein the thermal treatment is steam heat treatment, and even more particularly wherein the steam heat treatment comprises placing the vehicle into an autoclave or in a tank equipped with a heating jacket, and applying steam heat at a temperature within the range from 110 to 130 °C for a period of time from 2 minutes to 2 hours, and even more particularly wherein the suspending agent is carboxymethyl cellulose or a pharmaceutically acceptable salt thereof such as sodium carboxymethyl cellulose, and the vehicle further comprises a bulking agent which is mannitol, a buffering agent which is disodium phosphate monohydrate, and a pH adjusting agent which is sodium hydroxide;
c) combining a) and b) to form a primary suspension comprising aripiprazole in the form of particles, particularly further comprising homogenizing the primary suspension, more particularly wherein homogenizing is carried out with a high shear homogenizer at a speed from 15 to 40 m/s for at least 15 min;
d) reducing the size of the aripiprazole particles to obtain a secondary suspension, wherein the aripiprazole particles in the secondary suspension have a volume moment mean diameter D[4,3] from 1 to 10 microns, particularly wherein the reduction of the particle size comprises simultaneous milling and homogenization of the suspension of the previous step for one or more passes until the aripiprazole in the suspension has the desired particle size, more particularly wherein milling is bead milling and/or the milling is carried out from 3 to 30 passes, more particularly from 4 to 7 passes, and/or the homogenization is carried out with a high shear homogenizer at a speed from 15 to 40 m/s for at least 30 min;
e) optionally homogenizing the secondary suspension to form a final suspension, particularly wherein homogenizing is carried out with a high shear homogenizer at a speed from 15 to 40 m/s at least for 5 min;
f) optionally freeze drying the suspension obtained in the previous step to form a freeze-dried formulation, and
g) optionally reconstituting the freeze-dried formulation with water.

As mentioned above, the second aspect of the invention relates to a process for the preparation of the pharmaceutical composition comprising aripiprazole and a vehicle which comprises:
i) providing aripiprazole;
ii) providing a vehicle which is an aqueous solution;
iii) combining i) and ii) to form a primary suspension comprising aripiprazole;
iv) reducing the size of the aripiprazole particles to obtain a secondary suspension,
wherein the aripiprazole particles in the secondary suspension have a volume moment mean diameter D[4,3] from 1 to 10 microns, wherein the reduction of the particle size comprises simultaneous milling and homogenization of the suspension of the previous step until the aripiprazole in the suspension has the desired particle size.

The features of the bulk aripiprazole of step i) are as defined herein. In one embodiment, optionally in combination with one or more features of the various embodiments described above or below, the bulk aripiprazole is sterile.

The aqueous vehicle of step ii) comprises one or more excipients as previously defined, which have to be soluble in water in the amounts in which they are employed.

In one embodiment, optionally in combination with one or more features of the various embodiments described above or below, the vehicle of step ii) comprises one or more excipients selected from the group consisting of suspending agents, bulking agents, buffering agents, pH adjusting agents, and combinations thereof, more particularly wherein the excipients are present in amount such that they are solubilized in water, i.e. form an aqueous solution. The suspending agents, bulking agents, buffering agents, pH adjusting agents are as defined herein.

In one embodiment of the second aspect of the invention, optionally in combination with one or more features of the various embodiments described above or below, the vehicle comprises a suspending agent which comprises a cellulose, more particularly the cellulose is selected from the group consisting of carboxymethyl cellulose or a pharmaceutically acceptable salt thereof, hydroxypropyl cellulose, carboxymethyl cellulose, hydroxypropylethyl cellulose, hydroxypropylmethyl cellulose, and mixtures thereof, even more particularly, the cellulose is sodium carboxymethyl cellulose. In another embodiment, the suspending agent is carboxymethyl cellulose or a pharmaceutically acceptable salt thereof such as sodium carboxymethyl cellulose.

In one embodiment, optionally in combination with one or more features of the various embodiments described above or below, the vehicle comprises a suspending agent, a bulking agent, a buffering agent, a pH adjusting agent, and water. More particularly, the vehicle comprises a suspending agent which is carboxymethyl cellulose or a pharmaceutically acceptable salt thereof such as sodium carboxymethyl cellulose, a bulking agent which is mannitol, a buffering agent which is disodium phosphate monohydrate, a pH adjusting agent which is sodium hydroxide, and water. Even more particularly, the vehicle of the primary suspension comprises from 0.10 to 1.5% by weight of a suspending agent such as sodium carboxymethyl cellulose, from 0.5 to 10% by weight of a bulking agent such as mannitol, from 0.01 to 0.5% of by weight a buffering agent disodium phosphate monohydrate, from 85 to 99% by weight of water, and, if needed, a pH adjusting agent such as sodium hydroxide in an amount sufficient to adjust the pH of the formulation within the range from 6 to 7.5, wherein all percentages are with respect to the total weight of the vehicle, and wherein the sum of the percentages is equal to or lower than 100%.

The vehicle of step ii) may be sterilized. Any sterilization method known in the art, such as a filtration method or a thermal method like the ones described herein.

In one embodiment, optionally in combination with one or more features of the various embodiments described above or below, the vehicle of step ii) has been submitted to a thermal treatment, more particularly the thermal treatment is steam heat treatment, and even more particularly, the steam heat treatment comprises placing the vehicle into an autoclave, and applying steam heat at a temperature within the range from 110 to 130 °C for a period of time from 2 minutes to 2 hours. If desired, previously to or after the thermal treatment for sterilization, the vehicle may be additionally sterilized through a filter, typically a 0.22 micron filter. Thus, in one embodiment, optionally in combination with one or more features of the various embodiments described above or below, the vehicle is filtered through a filter, particularly a 0.22 micron filter, before being thermally treated or after that.

Step iii) of combining i) and ii) to form a primary suspension comprising aripiprazole may advantageously comprise further homogenizing the primary suspension as already mentioned for the process of the first aspect of the invention. Thus, in one embodiment, optionally in combination with one or more features of the various embodiments described above or below, step iii) further comprises homogenizing the primary suspension. More particularly, homogenizing is carried out with a high shear homogenizer at a speed from 15 to 40 m/s, for at least 15 min.

In one embodiment, optionally in combination with one or more features of the various embodiments described above or below, the primary suspension is sterile. In another embodiment, optionally in combination with one or more features of the various embodiments described above or below, the secondary suspension is sterile.

Analogously to the process of the first aspect of the invention, if desired, after step iv), a final homogenization step may be carried out. Thus, in one embodiment, optionally in combination with one or more features of the various embodiments described above or below, after step iv) the process further comprises step v) of homogenizing the secondary suspension to form a final suspension. More particularly, step v) is carried out with a high shear (rotor-stator) homogenizer at a speed from 15 to 40 m/s at least for 5 min.

In one embodiment, optionally in combination with one or more features of the various embodiments described above or below, the final suspension is sterile.

Furthermore, the secondary suspension obtained in step iv) or the final suspension of step v) may be finally freeze-dried to obtain a freeze-dried aripiprazole formulation, and the freeze-dried formulation may be further reconstituted with water for injection before being administered and is able to release a therapeutic effective amount of aripiprazole over a as described above in the first aspect of the invention.

In one embodiment of the second aspect, the invention relates to a process for the preparation of a pharmaceutical composition comprising aripiprazole and a pharmaceutically acceptable vehicle, wherein the process comprises:
i) providing aripiprazole, particularly aripiprazole in the form of particles having a volume moment mean diameter D[4,3] from 50 to 200 microns;
ii) providing a vehicle which is an aqueous solution, particularly wherein the vehicle has been submitted to a thermal treatment, more particularly wherein the thermal treatment is steam heat treatment, and even more particularly wherein the steam heat treatment comprises placing the vehicle into an autoclave or in a tank equipped with a heating jacket, and applying steam heat at a temperature within the range from 110 to 130 °C for a period of time from 2 minutes to 2 hours, and even more particularly wherein the vehicle comprises a suspending agent which is carboxymethyl cellulose or a pharmaceutically acceptable salt thereof such as sodium carboxymethyl cellulose, a bulking agent which is mannitol, a buffering agent which is disodium phosphate monohydrate, and a pH adjusting agent which is sodium hydroxide;
iii) combining i) and ii) to form a primary suspension comprising aripiprazole in the form of particles, particularly further comprising homogenizing the primary suspension, more particularly wherein homogenizing is carried out with a high shear homogenizer at a speed from 15 to 40 m/s for at least 15 min;
iv) reducing the size of the aripiprazole particles to obtain a secondary suspension, wherein the aripiprazole particles in the secondary suspension have a volume moment mean diameter D[4,3] from 1 to 10 microns, wherein the reduction of the particle size comprises simultaneous milling and homogenizing the suspension of the previous step until the aripiprazole in the suspension has the desired particle size, more particularly wherein milling is bead milling and/or the milling is carried out from 3 to 30 passes, more particularly from 4 to 7 passes, and/or the homogenization is carried out with a high shear homogenizer at a speed from 15 to 40 m/s for at least 30 min;
v) optionally homogenizing the secondary suspension to form a final suspension, particularly wherein homogenizing is carried out with a high shear homogenizer at a speed from 15 to 40 m/s at least for 5 min;
vi) optionally freeze drying the suspension obtained in the previous step to form a freeze-dried formulation, and
vii) optionally reconstituting the freeze-dried formulation with water.

All features and embodiments explained above in relation to the first aspect of the invention both referred to the process or the products obtained in each of the steps of the process also apply to the second aspect of the invention. In particular, the features of particle size and viscosity already mentioned in the first aspect of the invention form the primary suspension, the secondary suspension, the final suspension, the freeze-dried formulation, and the reconstituted suspension are the same than the ones obtained in the process of the second aspect of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### Example 1

### 1) Vehicle

Carboxymethyl cellulose sodium salt (Aqualon CMC 7L2P, 13.09 g) was slowly added to water for injection (WFI, 2448 g) and stirred for 1 hour to complete dissolution. Mannitol (65.46 g), disodium phosphate monohydrate (1.17 g) were then added and the mixture was stirred for 10 minutes. The resulting solution was made up with water for injection to total mass of 2770 g and pH was adjusted to pH 7 (10% w/v NaOH_{aq}, 3 mL). Afterwards the solution was filtered through a 0.22 micron filter and collected into a 5 L glass bottle. The bottle containing the filtrate was autoclaved in a standard cycle (121 °C for 15 minutes) and cooled to ambient temperature.

### 2) Primary suspension

Aripiprazole (327.24 g, D[4,3] = 86.5 microns) was mixed with the vehicle as prepared above in a high shear (rotor-stator) homogenizer (KappaVita HM5 from Netzsch) operating at 12000 rpm (31 m/s) of rotor speed for 1 hour. The temperature of the suspension during homogenization was around 30 °C (active cooling by means of a cooling jacket on the homogenizing vessel was maintained). After homogenization, the particle size of aripiprazole was 12 microns (D[4,3]) and Dv(100) was 75.8 microns.

### 3) Secondary suspension

The primary suspension was wet milled (LabStar LS1 from Netzsch, equipped with Mini milling chamber of 120 mL nominal volume). Milling was done in recirculation mode: the suspension circulated between the homogenizer and the milling chamber (mill tip speed: 1000 rpm, flowrate: 140 g/min). The suspension was fed to the milling chamber by peristaltic pump. The homogenizer (the same as used for preparation of the primary suspension) was operating at 10000 rpm of rotor speed. Milling was performed with zirconium oxide, yttrium stabilized milling beads of 0.5 mm in diameter. Milling bead load was 45% of nominal milling chamber volume. Milling-homogenization was performed for 6.0 theoretical passes. The temperature of the secondary suspension during milling was around 15 °C (the temperature of cooling water was 6 °C). During milling particle size and particle size distribution (PSD) was monitored using a Malvern Mastersizer 3000 particle size analyzer.

After 6.0 theoretical passes of suspension through the milling chamber, the particle size (D[4,3]) of aripiprazole was around 4.40 microns (Dv(10) = 1.15 microns, Dv(50) = 3.27 microns, Dv(90) = 8.54 microns, Dv(99) = 22.4 microns).

### 4) Final suspension

After milling was stopped, the suspension flow between milling chamber and homogenizer was closed. A final homogenization step was performed at 12000 rpm (31 m/s) of rotor speed for 10 minutes. The temperature of the suspension during homogenization was around 30 °C. After homogenization, the particle size (D[4,3]) of aripiprazole was around 4.57 microns (Dv(10) = 1.12 microns, Dv(50) = 3.28 microns, Dv(90) = 8.72 microns, Dv(99) = 27.6 microns).

### 5) Lyophilization (freeze drying)

The suspension was dispensed into glass vials (R8), partially stoppered with lyophilization-compatible stoppers and put into lyophilizer (Epsilon 2-6D LSCplus from Martin Christ). The suspension was frozen to -35 °C over 0.5 h and kept at -35 °C for 1h, annealing step was performed at -18 °C. Next, the primary drying was performed for at least 35h. The shelf temperature during primary drying step was -10 °C or below, chamber pressure was 25 Pa. No secondary drying was performed. Afterwards the drying chamber was filled with nitrogen, the vials were stoppered and removed from the lyophilizer.

### Example 2

### 1) Vehicle

The vehicle was prepared as described in example 1 above using the following amounts of excipients: water for injection: 1949.8 g, carboxymethyl cellulose sodium salt: 10.91 g, mannitol: 54.55 g, disodium phosphate monohydrate: 0.983 g. The resulting solution was made up with water for injection to total weight of solution: 2308.3 g. Vehicle prepared in this Example differed from that described in example 1 in that it was filtered through a 0.22 micron filter and was not autoclaved.

### 2) Primary suspension

A primary suspension was prepared as described in Example 1 using aripiprazole (272,70 g, D[4,3] = 186 microns). The operating conditions were 12000 rpm (31 m/s) of rotor speed for 30 minutes. After homogenization, the particle size of aripiprazole was around 11.7 microns (D[4,3]) and Dv(100) was 83.8 microns.

### 3) Secondary suspension

The primary suspension was wet milled (LabStar LS1 from Netzsch, equipped with Mini milling chamber of 120 mL nominal volume). Milling was done in recirculation mode: the suspension circulated between the mill and the steel vessel with mechanical agitator. Milling was performed with zirconium oxide, yttrium stabilized milling beads of 0.8 mm in diameter. Milling beads' load was 60% of nominal milling chamber volume. Mill tip speed: 1000 rpm, flowrate: 130 g/min. Milling was performed for 6.5 theoretical passes.
The temperature of the secondary suspension during milling was around 15 °C (the temperature of cooling water was 6 °C). During milling particle size and PSD was monitored using a Malvern Mastersizer 3000 particle size analyzer.
After 6.5 theoretical passes of suspension through the milling chamber, the particle size (D[4,3]) of aripiprazole was around 4.98 microns (Dv(10) = 1.33 microns, Dv(50) = 3.79 microns, Dv(90) = 10.2 microns, Dv(99) = 20.3 microns).

### 4) Final suspension

After milling was stopped, the suspension flow between milling chamber and homogenizer was closed. A final homogenization step was performed at 12000 rpm (31 m/s) of rotor speed for 10 minutes. The temperature of the suspension during homogenization was around 30 °C. After homogenization, the particle size (D[4,3]) of aripiprazole was around 4.21 microns (Dv(10) = 1.21 microns, Dv(50) = 3.31 microns, Dv(90) = 8.43 microns, Dv(99) = 15.8 microns).

### 5) Lyophilization (freeze drying)

The suspension obtained after the milling step was dispensed into glass vials and lyophilized as described in example 1.

### Comparative example 3

The product was prepared as described in Example 2 with the difference that the secondary suspension obtained after the milling-homogenization step was directly lyophilized according to lyophilization cycle described in Example 1 without being subjected to an additional homogenization (step 4 of Example 2). After 6.5 theoretical passes of suspension through the milling chamber, the particle size (D[4,3]) of aripiprazole was around 4.98 microns (Dv(10) = 1.33 microns, Dv(50) = 3.79 microns, Dv(90) = 10.2 microns, Dv(99) = 20.3 microns).

### Particle size analysis

In FIG. 1 the particle size decrease (Dv(50)) during the milling process is shown. During the milling process samples of the suspension circulating between milling chamber and homogenizing tank (example 1) or steel vessel with mechanical agitator (comparative example 3) were collected and analysed with Laser Diffraction technique (Mastersizer 3000 from Malvern).

Empty circles denote batch prepared according to comparative example 3. Full black circles denote batch prepared according to example 1. Interpolation of datapoints was done with negative exponential function. It can be observed that the milling process according to comparative example 3 is apparently slower and less effective than the process according to example 1. The PSD observed by Laser Diffraction shows aggregates formed in the milling chamber and the holding tank together with primary particles. Slower milling progress is caused by sub-optimal use of the kinetic energy of the beads which crush not only primary particles but also secondary aggregates. This effect can be detrimental for quality of the suspension since PSD monitored during milling and recorded afterwards is not reflecting actual primary particle sizes, which are effectively smaller.

On the other hand, the process according to example 1 is more effective since markedly smaller particles are produced in fewer theoretical passes of suspension through the milling chamber. This observation leads to the conclusion that it is possible to use lower kinetic energy of the beads to obtain PSD change kinetics comparable with traditional process. The reason of this overall increase in effectiveness of milling is usage of homogenization during milling to disrupt the aggregates formed during milling. Since homogenization is done online, one can observe primary particles on each milling stage.

Viscosity of the product obtained in a process without final homogenization and without vehicle thermal treatment (comparative example 3) is 16.4 mPa*s while final homogenization (example 2) allows to decrease viscosity of the product to 14.7 mPa*s. This observation also suggests presence of agglomerates in non-homogenized product.

### Stability study

After freeze drying was complete, vials of examples 1, 2 and the vials of the comparative example 3 were stored at 40 °C/75%RH for 3 months. At predefined timepoints samples were withdrawn from climatization and analysed for viscosity after reconstitution. Reconstitution procedure used was as follows: 1.9 mL water for injections was added to a vial containing lyophilizate and the vial was vigorously shaken for at least 30 seconds. Resulting suspension was withdrawn from the vial with a syringe. Timepoints at which samples were analysed were: 0 months (before climatization), 1 month (3 weeks for batch according to example 1), 2 months, 3 months. Samples withdrawn from climatization were reconstituted with water for injection to form suspension with active pharmaceutical ingredient (API) concentration of 200 mg/mL on anhydrous substance basis. Viscosity of reconstituted suspensions was measured using Brookfield type viscometer.
In FIG. 2 Viscosity of reconstituted suspension after climatization is shown for batches according to example 1 and 3.

It is evident that that batch prepared according to example 1 (black circles) shows markedly lower initial viscosity compared to the batch prepared according to comparative example 3 (empty circles). Also, significant and unacceptable viscosity change is observed for the latter while no significant change is observed for the former. Increase of viscosity in time is a result of aggregation, both initial and progressing in solid state, of particles in batch prepared without thermal treatment and homogenization of suspension during milling (comparative example 3). No such effect is observed once thermal treatment of the vehicle and homogenization during milling is employed.
Hence, these results suggest that deagglomeration of the particles by means of the homogenization only after milling process is not sufficient to effectively stabilize the suspension.

Moreover, it is generally recognized that carboxymethyl cellulose sodium salt is negatively affected by thermal treatment in temperatures above 100 °C in a way which results in permanent viscosity loss due to hydrolysis of polymeric chains. However, according to the process described herein no significant change in molecular weight, as measured by SEC-MALS was observed, only a minor change in viscosity (ca. 5 %) was noted. No correlation between the time of heating and viscosity or molecular weight was observed, which indicates no significant hydrolysis of carboxymethylcellulose sodium salt chains due to thermal treatment. Once in solution the carboxymethylcellulose sodium salt chains stabilize API particles through electrostatic repulsion. Effectiveness of interaction with API particles and stabilizing effect of repulsion is greater when polymeric chains are untangled and not agglomerated. This effect is clearly visible on FIG. 2. Increase of viscosity upon storage is caused by API particles' aggregation in lyophilizate presumably due to insufficient stabilization by polymer and formation of mixed agglomerates of API and polymer (as confirmed by microscopic analysis). Such aggregation was not observed in batch according to example 1.

### Citation List

WO2005041937
WO2009017250
WO2012169662
WO2003026659

## Claims

1. A process for the preparation of a pharmaceutical composition comprising aripiprazole and a pharmaceutically acceptable vehicle, wherein the process comprises:
a) providing aripiprazole;
b) providing a vehicle which is an aqueous solution, wherein the vehicle comprises a suspending agent which comprises a cellulose, and wherein the vehicle has been submitted to a thermal treatment;
c) combining a) and b) to form a primary suspension comprising aripiprazole in the form of particles; and
d) reducing the size of the aripiprazole particles to obtain a secondary suspension, wherein the aripiprazole particles in the secondary suspension have a volume moment mean diameter D[4,3] from 1 to 10 microns.

2. The process according to claim 1, wherein the aripiprazole of step a) is in the form of particles having a volume moment mean diameter D[4,3] from 50 to 200 microns.

3. The process according to any of claims 1-2, wherein the vehicle further comprises one or more excipients selected from the group consisting of bulking agents, buffering agents, pH adjusting agents, and combinations thereof.

4. The process according to any of claims 1-3, wherein the suspending agent is carboxymethyl cellulose or a pharmaceutically acceptable salt thereof, and the vehicle further comprises a bulking agent which is mannitol, a buffering agent which is disodium phosphate monohydrate, and a pH adjusting agent which is sodium hydroxide.

5. The process according to any of claims 1-4, wherein the thermal treatment is steam heat treatment or heat treatment in a tank equipped with a heating device.

6. The process according to claim 5, wherein the steam heat treatment comprises placing the vehicle into an autoclave, and applying steam heat or placing the vehicle into a tank equipped with a heating device at a temperature within the range from 110 to 130 °C for a period of time from 2 minutes to 2 hours.

7. The process according to any of claims 1-6, wherein step c) further comprises homogenizing the primary suspension.

8. The process according to claim 7, wherein homogenizing is carried out with a high shear homogenizer at a speed from 15 to 40 m/s for at least 15 min.

9. The process according to any of claims 1-8, wherein the reduction of the particle size comprises simultaneous milling and homogenization of the suspension of the previous step for one or more passes until the aripiprazole in the suspension has the desired particle size.

10. The process according to claim 9, wherein milling is bead milling.

11. The process according to claim 10, wherein the milling is carried out from 3 to 30 passes.

12. The process according to any of claims 9-11, wherein the homogenization is carried out with a high shear homogenizer at a speed from 15 to 40 m/s for at least 30 min.

13. The process according to any of claims 1-12, wherein after step d) the process further comprises:
e) homogenizing the secondary suspension to form a final suspension.

14. The process according to claim 13, wherein step e) is carried out with a high shear homogenizer at a speed from 15 to 40 m/s at least for 5 min.

15. The process according to any of claims 1-14, wherein the process further comprises freeze drying the suspension obtained in the previous step to form a freeze-dried formulation.
